# EUROPEAN PATENT APPLICATION

(11) **EP 1 798 284 A1**
(43) Date of publication of application: **20.06.2007**
(21) Application number: 05077869.5
(22) Date of filing: 15.12.2005
(51) Int. Cl.: C12N 11/14, C09K 8/582

(54) **Immobilisation of bacteria to a geological material**

(71) Applicant: Stichting Geodelft, 2628 CK Delft (NL)
(72) Inventor: van Paassen, Leon Andreas, 2613 WD Delft (NL); Whiffin, Victoria Suzanne, 2611 JE Delft (NL); Harkes, Marinus Pieter, 3061 VM Rotterdam (NL)
(74) Representative: Winckels, Johannes Hubertus F.

(57) **Abstract**

The invention relates to a method for immobilising bacteria in a material permeable to the bacteria, in particular a geological body, comprising forming a first zone comprising the bacteria in the material and allowing the first zone to move through at least part of the material; forming a second zone comprising an effective amount of a flocculating agent to the material and allowing the second zone to move through at least part of the material,
wherein the zones are allowed to move such that the zones become at least partially overlapping and at least part of the bacteria flocculate, thereby becoming immobilised.

## Description

The invention relates to a method for immobilising bacteria in a material, in particular a geological body.

It is known that specific naturally present bacteria can cause biomineralisation in natural settings. Based upon this knowledge, the use of bacterial urease to modify a porous medium has been described by Nemati and Voordauw in Enzyme and Microbial Technology 33 (2003), page 635-642.

It has been contemplated that bacteria may be used without having to isolate the urease from the bacteria. The inventors have found that by merely flowing the bacteria and substrate through a material to be treated unsatisfactory results are achieved. In particular clogging tends to occur due to the particles forming outside of the material (soil) that is treated. As a result the material looses permeability for the substrate and bacteria.

The inventors have found that it is possible to immobilise bacteria in the material in a specific way, such that the immobilised bacteria can be used to modify the material, in particular by allowing the bacteria to form a mineral.

Accordingly, the present invention relates to a method for immobilising bacteria in a material permeable to the bacteria, in particular a geological body, comprising
forming a first zone comprising the bacteria in the material and allowing the first zone to move through at least part of the material;
forming a second zone comprising an effective amount of a flocculating agent to the material and allowing the second zone to move through at least part of the material,
wherein the zones are allowed to move such that the zones become at least partially overlapping and at least part of the bacteria flocculate, thereby becoming immobilised.

The above steps may be repeated once or more often to increase the amount of immobilised bacteria (and thereby increasing the bacterial activity per volume of material)

It has been found that by a method according to the invention, the material can be strengthened in a controlled manner by allowing the bacteria to form a mineral in situ, whilst avoiding clogging of the pores in the material. Accordingly, in situ mineral deposition can be realised efficiently over a long distance of the material.

A method according to the invention has been found effective in immobilising the bacteria such that their availability to perform a material modifying activity is improved. In particular, it has been found that immobilisation is advantageous prior with respect to biomineralisation by the bacteria in the material.

A method according to the invention may in particular be used in civil engineering or mining. It may be used to modify soil or another geological formation, in particular to stabilise and/or fortify the material. This has been found possible whilst maintaining a satisfactory porosity of the material, such as a geological body, to allow treatment of the material over a long distance.
Figure 1 shows the effectiveness of several flocculation agents in causing flocculation of bacteria in a suspension.
Figure 2 shows a profile of the two zones moving through the material.
Figure 3 shows the location of immobilised bacteria in a sand material.
Figure 4 shows urease activity at various locations in the material after immobilising the bacteria.
Figure 5 shows the effect of the formation of calcium carbonate on the porosity of the material wherein bacteria are immobilised.

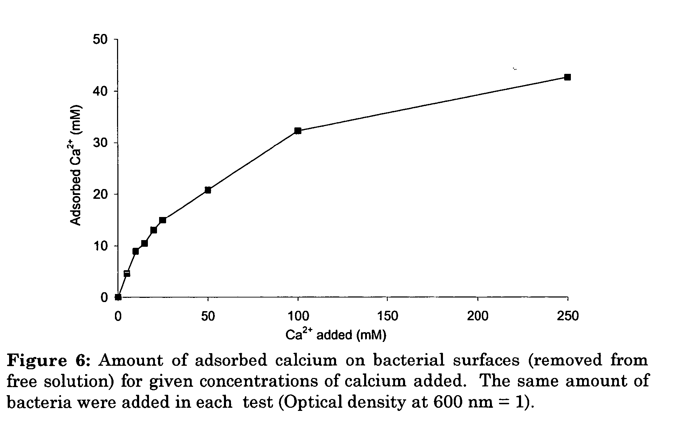

Figure 6 shows the effect of the adsorption of calcium as a function of added calcium ions.
Figure 7 shows the effect of the concentration of calcium chloride on the size of the bacterial flocs.

In principle any material that is permeable to the bacteria (i.e. any material that has voids that are sufficiently large to allow bacteria to pass through it, at least under pressure) may be used. In case it is desired to use the bacteria for an *in situ* activity such as biomineralisation, the material should usually also be permeable to the substrate for such activity (ions). A material to be used in a method of the invention is usually a geological formation or a mineral material. The geological formation may in particular be a body , comprising sediment (clay, silt, sand and/or gravel), sedimentary rock, soil material and/or rock material with permeability e.g. fractured rocks. Preferred materials include materials selected from the group consisting of silt, clay, sand and gravel bodies. Particularly good results have been achieved with sand.

In principle the method is suitable for immobilisation of any bacteria that are able to be flocculated with a suitable flocculation agent, in particular with divalent cations. The bacteria are preferably selected from bacteria that are capable of forming a mineral, such as CaCO₃. Preferably the bacteria are selected from the group consisting of urease producing bacteria, more preferably from the group consisting of *Sporosarcina,* in particular *Sporosarcina pasteurii.* Such bacteria have been found particular effective, because their ureases show good activity, also in the presence of ammonium (formed during biomineralisation).

The bacteria are usually added to the material in a concentration of at least 0.1, preferably 0.1 to 1.0, more preferably up to 0.5 mS/min. For a high bacterial activity (such as high biomineralisation rate after addition of a substrate), the bacteria are preferably added in a concentration of at least 0.3 mS/min (as determined by the relative change in electric conductivity produced when bacteria are added to 1 M urea at 20°C).

As a flocculating agent any agent may be used which under the existing conditions is effective in causing the bacteria to flocculate. Particularly suitable are divalent cations, in particular for immobilising urease producing bacteria such as *Sporosarcina* bacteria.

It is contemplated that the floccs are at least primarily formed as a result of bacteria and the cations (such as calcium) interacting together (ionic surface interaction) rather than as a result of precipitated mineral comprising the cation (such as a calcium carbonate salt) This insight is based on experiments wherein the floccs were treated with 2M HCI. No gas (CO₂) evolved.

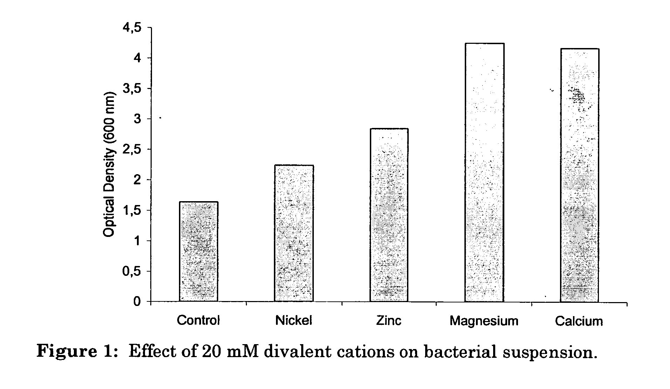

Preferably the divalent cation is selected from the group consisting of nickel-, zinc-, magnesium- and calcium ions. Magnesium and calcium ions are particularly preferred, because of their large effect on the flocculation (see also Figure 1).

An effective amount of the flocculating agent can be determined empirically. Usually, in particular for divalent cations, the concentration is at least 3 mM, preferably at least 5 mM.. Flocs with a relative large size are advantageous for obtaining a strong fixation of the bacteria, in particular in the case relatively coarse materials, such as coarse sand or gravel are treated. Accordingly, for a strong fixation it is preferred to use a concentration of at least 10 mm, in particular of at least 20 mM.

Usually the concentration of flocculation agent is up to about 1000 mM. The concentration is preferably up to 100 mM, in view of efficiency with respect to absorption to the surface of the bacteria.

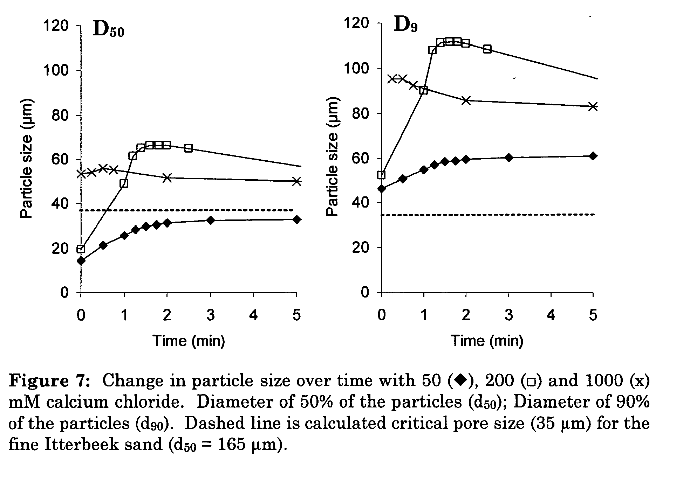

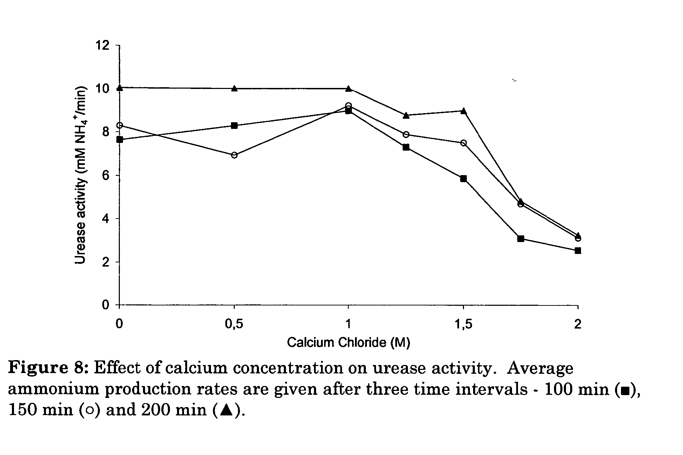

In addition, the size of the bacterial flocs may be set by choosing the concentration of flocculation agent (see e.g. Figure 7). For producing relatively small flocs, the concentration of flocculation agent (in particular calcium), is preferably 50 mM or less, more preferably 40 mM or less. This is advantageous in view of avoiding clogging, especially when the material is a finer sand, a clay or a silt.

A suitable way to move the zones in the material may be based on known methodology for introducing inorganic solutions into and moving them through the material. See *e.g.* WO 00/31209.

The zones may be moved by making use of hydraulic pumping and/or electrokinetically. Hydraulic pumping is in particular preferred for a relatively coarse material such as sand or a material that has at least the same average particle size. Electrokinetic migration is in particular preferred for relatively low permeability materials such as clay or silt.

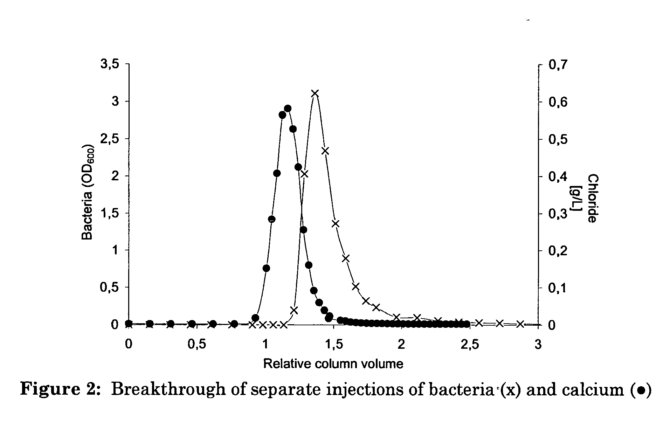

In a preferred method the second zone is formed directly after the first zone and both zones are allowed to move in the same direction through at least part of the material. This may suitably be accomplished by first adding (for instance by injection) a liquid comprising the bacteria to the material and thereafter a liquid comprising the flocculating agent. Due to the particulate nature and/or surface charge of the bacteria, the bacteria typically move through the material at a lower velocity than the flocculating agent. Thus, as both zones move through the material the zones will (partially) overlap and the flocculating agent gets in contact with the bacteria, thereby causing the bacteria to flocculate. The flocs are typically too large to move through the material and are thereby immobilised. Figure 2 shows and example of the velocity of a suspension of the bacteria and a liquid comprising the flocculating agent in a column comprising sand. It illustrates that the bacteria move slower than the flocculating agent and that the zone with the flocculating agent (visualised by the left peak) mixes with the zone with the bacteria (visualised by the right peak).

Conveniently, bacteria and flocculation agent are moved in the same direction. It is also possible to cause mixing by moving the zone comprising bacteria and the zone comprising flocculation agent under an angle, for instance the bacteria could be moved essentially horizontally and the flocculation agent essentially vertically or under an angle between 0 and 90 ° relative to the bacteria, or vice versa. In principle the bacteria and flocculation agent may be moved in essentially opposite directions, toward each other (e.g. electrophoretically/electrokinetically optionally in combination with pumping).

The liquid comprising the bacteria is preferably added at an extremity of the material to be treated at least until the bacteria can be detected at the opposite extremity of the material. Detection can suitably be done by monitoring the optical density, *e.g.* at 600 nm, by microscopic verification by visual observation or another technique known in the art, at the opposite extremity.

The liquid comprising the flocculating agent is preferably added to the material at least until the flocculating agent or another component of this liquid is detectable at the opposite extremity. This can be done by any suitable detection technique for the component to be detected. Monitoring of conductivity is generally a suitable way.

The immobilisation may be carried out under ambient conditions, in particular ambient temperature.

The bacteria and the flocculating agent are usually introduced into the material at a flow rate that is at least equivalent to a hydraulic gradient of 0.1 to 2.0. For practical reasons the hydraulic gradient is usually up to 0.8 to 1.2, preferably up to 1.0.

After the bacteria have been immobilised, a substrate may be added to the material to allow the bacteria to perform a material modifying activity, such as the generation of a mineral. The choice of substrate depends on the bacterium and its intended activity. For forming a mineral such as a carbonate, making use of bacteria having urease activity, urea and a cation, such as calcium can be added to cause biomineralisation. The concentration of the substrate ingredients may be chosen based upon methodology known in the art. Preferably the cation (calcium) concentration is at least 100 mM, more preferably in the range of 800 to 1500 mM. The urea concentration is preferably (about) equimolar to the concentration of the added cation.

The invention will now be illustrated by the following examples.

### Example 1: flocculation of bacteria

A suspension of *Sporosarcina pasteurii* bacteria (Optical density at 600 nm = 1.0) was mixed with a salt of several divalent cations at a temperature of 20°C to a final concentration of 20 mM of the divalent cation. The salts were Ni(NO₃)₂, ZnCl₂, Mg(NO₃)₂ respectively CaCl₂.

After 5 min. of mixing the optical density at 600 nm was determined and compared with a control (bacteria suspension without added divalent cations). Figure 1 shows the results.

From the increase in optical density it can be conclude that nickel, zinc, magnesium and calcium are effective in causing flocculation.

### Example 2: determination of breakthrough volumes

A column (circular ID of 1.6 cm, 1 ) was filled with Itterbeek sand (d₅₀ = 420 µm) to a density of 1.7 g/cm³.

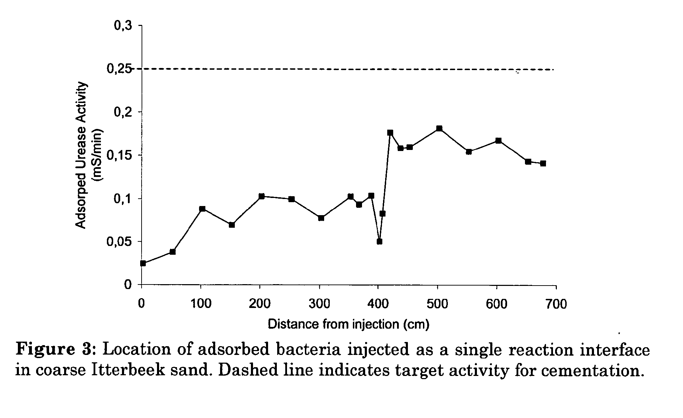

10 ml pulse of 50 mM calcium chloride was injected. Thereafter the calcium chloride was pumped through the column with deionised water and chloride levels were monitored at the end or the column. Figure 3 shows that the calcium chloride migrates through the material without substantially being delayed: the front of the calcium chloride zone reaches the end of the column after about one column volume and within 1.5 column volumes substantially all calcium chloride has migrated through the material.

In a separate experiment a suspension of *Sporosarcina pasteurii* bacteria (optical density at 600 nm of 4.2) was injected as a 10 ml pulse. The experiment shows that the front of the zone comprising the bacteria reaches the end of the column after more than one column volume has been passed through the column, indicating that the bacteria are migrating more slowly through the column than the flocculating agent (calcium chloride).

### Example 3: immobilisation of bacteria

A column (1.8 cm circular diameter, 6.8 m length) was filled with Itterbeek sand (d₅₀ = 420 µm) and maintained under saturated conditions. Bacteria (concentration = 0.5 mS/min) were injected until they appeared at the column outlet. Immediately after, the same volume of 50 mM calcium chloride was injected and allowed to stand for 1 hour. The column was then flushed with three times the pore volume of deionised water and cut into sections for urease activity analysis.

Urease activity was determined by weighing a known volume of sand into a container and adding urea to a final concentration of 1 M, then monitoring electric conductivity to obtain the urea hydrolysis rate (mS/min).

Urease activity was detected at all locations along the 6.8 m length of the column (Figure 3).

### Example 4: ex-situ cementation

A column (1.8 cm circular diameter, 6.8 m length) was filled with Itterbeek sand (d₅₀ = 420 µm) and maintained under saturated conditions. Bacteria (concentration = 0.5 mS/min) were injected until they appeared at the column outlet. Immediately after, the same volume of 50 mM calcium chloride was injected and allowed to stand for 1 hour. The column was then flushed with 3 x the pore volume of deionised water and cut into sections for urease activity analysis. Urease activity was determined as in example 3.

In addition to this test, column sections that were immediately next to the sections analysed for urease activity, were removed and placed in sealed vessles containing 1 M urea and 1 M calcium chloride to simulate the in-situ cementation. These vessels were gently mixed and ammonium production was monitored.

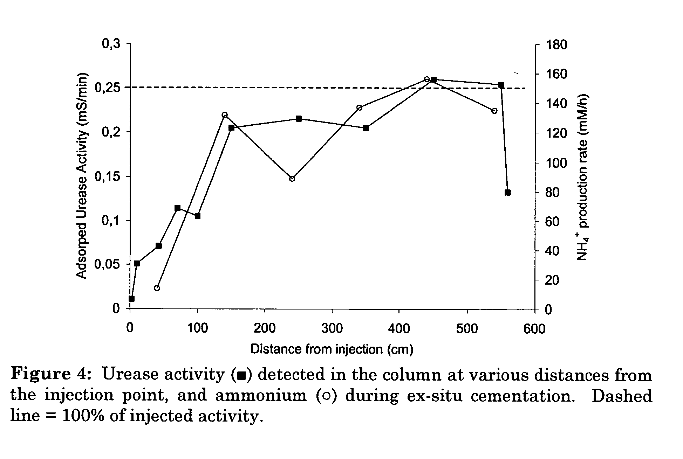

All sections of the core approached full cementation, except the section nearest the injection point (40cm). The urease activity detected in the core and the initial ammonium production rates (during the first 24 hours) were consistent (Figure 4).

### Example 5: Effect of CaCO₃ content on porosity

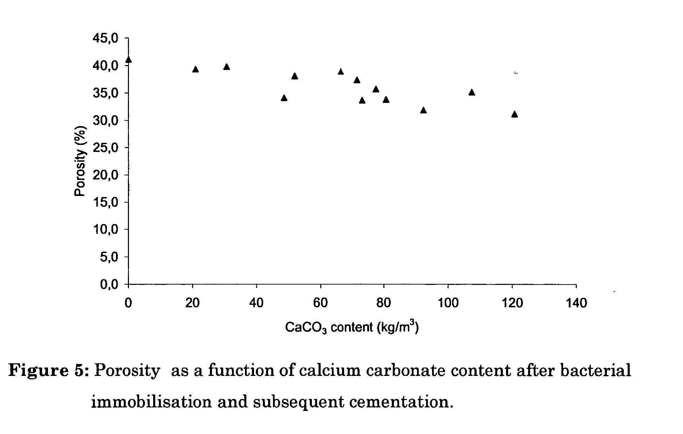

The experiment of Example 4 was repeated in an upscaled column (I.D. 6.6 cm; Length 5 m) with a hydraulic gradient of 0.3. 1.1 M equimolar urea and calcium chloride solution was injected and cemented in a gradient along the column to obtain different CaCO₃ contents in the material. The column was cut into sections and the effect on the porosity was determined. As shown in Figure 5 only a minor porosity decrease was observed with increasing calcium carbonate content after bacterial immobilisation with the given method and subsequent cementation. This supports that the invention is suitable to cause biomineralisation in bulky materials or over a long distance in geological formations.

### Example 6: Effect of flocculation agent concentration on floc size

An amount of bacteria (Optical density at 600 nm = 1.0) was mixed with various CaCl₂ concentrations. The effect on the floc size was determined with laser diffraction. As shown in Figure 7. The floc size increases with increasing concentration of flocculation agent.

## Claims

1. Method for immobilising bacteria in a material permeable to the bacteria, in particular a geological body, comprising
forming a first zone comprising the bacteria in the material and allowing the first zone to move through at least part of the material;
forming a second zone comprising an effective amount of a flocculating agent to the material and allowing the second zone to move through at least part of the material,
wherein the zones are allowed to move such that the zones become at least partially overlapping and at least part of the bacteria flocculate, thereby becoming immobilised.

2. Method according to claim 1, wherein the second zone is formed directly after the first zone and both zones are allowed to move in the same direction through at least part of the material.

3. Method according to claim 1 wherein the first zone is moved in a first direction and the second zone under an angle from the first direction, wherein the direction is such that the zones move towards each other and cross each other.

4. Method according to any one of the preceding claims, wherein the material is a material selected from the group consisting of geological bodies comprising sediment (such as clay, silt, sand and/or gravel); sedimentary rocks; soil materials and rock material permeable to bacteria (such as fractured rocks).

5. Method according to any one of the preceding claims, wherein the flocculating agent is selected from the group consisting of divalent cations, preferably from the group consisting of nickel-, zinc-, magnesium- and calcium ions.

6. Method according to any one of the preceding claims, wherein the flocculating agent is added to the material at a concentration of 3 to 100 mmol/l liquid.

7. Method according to any one of the preceding claims, wherein the bacteria are selected from the group consisting of urease producing bacteria, preferably selected from the group consisting of *Sporosarcina,* in particular *Sporosarcina pasteurii.*

8. Method according to any one of the preceding claims, wherein the bacteria are added to the material in the form of a suspension comprising 0.1 to 1.0 mS/min (as determined by the relative change in electric conductivity produced when bacteria are added to 1 M urea at 20°C).

9. Method according to any one of the preceding claims, wherein the first and/or the second zone are moved through the material at a rate defined by a hydraulic gradient in the range of 0.1 to 2.0.

10. Method according to any one of the preceding claims, wherein after immobilisation a substrate is added to the material in an effective amount to allow the bacteria to form a mineral.

11. Method according to claim 10, wherein urea and calcium ions are brought into contact with the immobilised bacteria in effective amounts to allow the bacteria to form CaCO₃.
